# EUROPEAN PATENT APPLICATION

(11) **EP 2 608 089 A1**
(43) Date of publication of application: **26.06.2013**
(21) Application number: 11306751.6
(22) Date of filing: 22.12.2011
(51) Int. Cl.: G06F 19/00

(54) **Method to transfer personal health information of a subscriber**

(71) Applicant: Gemalto SA, 92190 Meudon (FR)
(72) Inventor: Bradley, Paul, 92190 MEUDON (FR)

(57) **Abstract**

The invention relates to a method for transferring in-case-of-emergency (ICE) information from a first device (1) of a first user to a second device (2) of a second user characterized in that it comprises transferring said information with a near range communication.

## Description

### FIELD OF THE INVENTION

The present invention relates generally to an ICE (In Case of Emergency) application and more specifically to a method for accessing to ICE information from a first device of a first user to a second device of a second user.

### BACKGROUND OF THE INVENTION

The emergency contact process may take a long time to identify a person in an accident when unconscious, and to research a next of kin or health information. A known application called ICE (In Case of Emergency) is used for the storage on a mobile device of critical personal and medical information comprising the name, contact, telephone numbers, blood type, allergies, medications prescribed, existing medical conditions, health insurance provider information...

Such an application is useful for example in case of an accident for a first responder such as emergency medical staff to retrieve the patient's health information and emergency contacts via a mobile device in peer-to-peer mode, a portable contactless reader in card emulation mode or a terminal device, e.g. a personal laptop, a desktop computer, a tablet or any digital system running an operating system hosting applications.... This application allows the retrieval of medical information even if the subscriber lies unconscious.

However the contact information and health information are not necessarily readily accessible with the existing ICE application when the device is locked with a user password or PIN screen.

There is then a need for accessing such information very quickly and easily as the speed of intervention by the medical crew may be determining.

It is then an object of the invention to provide access to this information in a quick and easy manner.

Thereto, the present invention provides a method for transferring in-case-of-emergency (ICE) information from a first device of a first user to a second device of a second user characterized in that it comprises transferring said information with a near range communication.

According to another aspect of the invention, the method may comprise storing said information securely in said first device by the first user, said information being related to said first user and encrypted with the public key of the second user; fetching said encrypted information by/with the second device over the near range communication.

According to another aspect of the invention, said information may comprise name, medical history, allergies, vaccinations, blood type and/or emergency contact information related to said first user.

According to another aspect of the invention, the information may be signed with the public key of the second user.

According to another aspect of the invention, the second user may be an authorized authority or works for an authorized authority, said authorized authority being a health authority or other government entity needing access to ICE information.

According to another aspect of the invention, the method may comprise transferring said information over NFC, Bluetooth, zigbee, or Wi-Fi.

According to another aspect of the invention, the method may comprise using a secure element supporting contactless data exchange for storing said ICE information into the first device.

According to another aspect of the invention, the method may comprise using an embedded secure element or an NFC-capable UICC, or a MicroSD as secure element.

According to another aspect of the invention, the method may comprise using a contactless reader or a mobile phone device as second device.

According to another aspect of the invention, upon receiving the encrypted information, the second device may apply its private key to said information and may decrypt said first information.

Thanks to the invention, it is advantageously possible to access and to transfer faster, simply and rapidly such information which could save a life.

Another advantage of the invention is that such information may be transferred securely. The first user can then decide which ICE information may be available unsecurely and which ICE information may need a secure transfer.

The various aspects, features and advantages of the invention will become more fully apparent to those having ordinary skill in the art upon careful consideration of the following Detailed Description, given by way of example thereof, with the accompanying drawings described below:
**FIGURE.1** schematically shows an embodiment of the present invention.
**FIGURE.2** schematically shows flowchart diagram of a step of the method according to the invention.

### DETAILED DESCRIPTION

The present invention may be understood according to the detailed description provided herein.

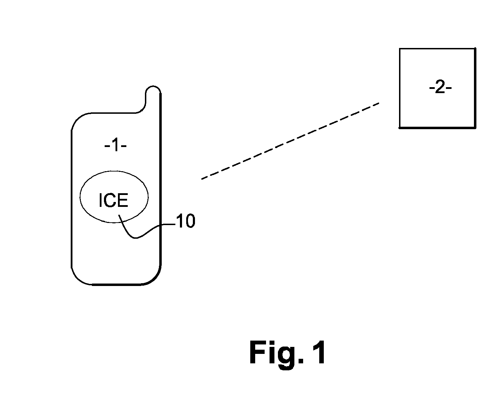

Shown in Figure 1 is a first device 1 of a first user, such as a mobile phone, comprising an embedded secured element 10 such as an NFC-capable UICC, a MicroSD or any other type of secure element supporting contactless or wireless data exchange or integrated circuit providing access to data over a personal area network.

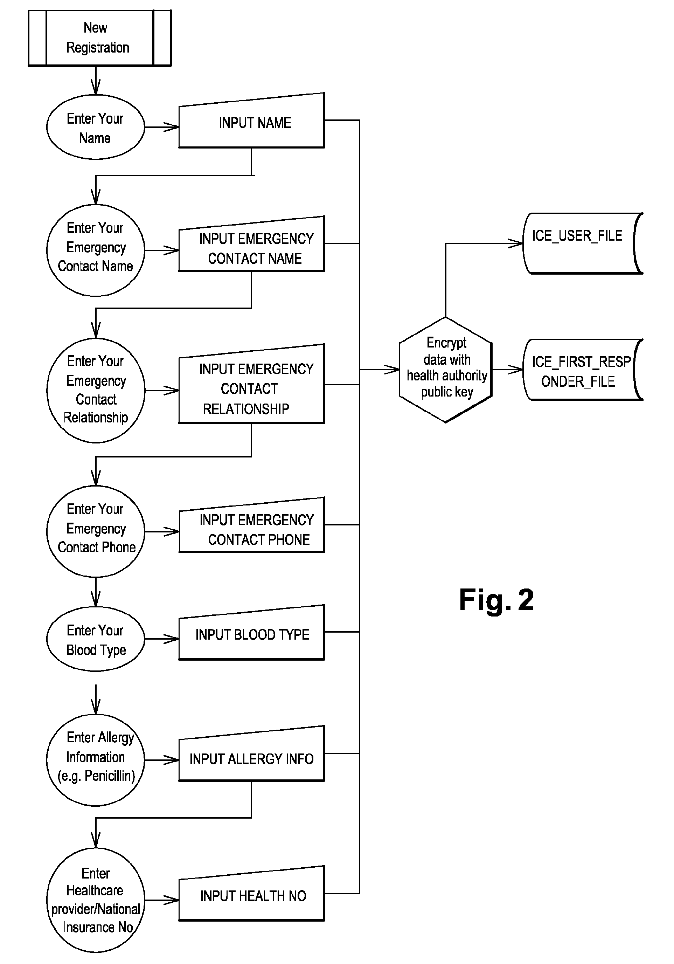

The first device 1 comprises the In-Case-of-Emergency application as decribed above. According to the present invention the method comprises a registration step wherein the first user registers information related to himself/herself. During this registration step, when launching the application for example for the first time, the first user sees a prompt to input information which comprises medical history, allergies, vaccinations, blood type and/or emergency contact information related to him as represented in figure 2.

It will be well understood that other data related to the first user can be stored. The first user can for example optionally also store information on his/her medical insurance.

The method comprises using the secure element 10 for storing said ICE information into the first device.

The information related to the first user is stored securely, i.e. encrypted with the public key of a second user or delivered to a second user by an authorized authority. An authorized authority is for example a health authority or other government entity needing access to such information.

Thus in the following description, a second user has to be understood as a health authority or someone who works for a health authority, an authorized authority or someone who works for the authorized authority.... The second user is then someone authorized by an authorized authority to access ICE information.

The health authority is for example entitled to deliver a public key for the encryption of the ICE information. The second user is then authorized by this health authority to access to information related to ICE information of the first user 1. He may be for example a member of an emergency team, or a member of the police or a firefighter, ... as long as such persons are authorized by the authorized authority for example by being delivered the private key necessary for the decryption of the ICE information stored into the first device 1 of the first user.

Optionally the information is signed with the public key of the second user.

The method comprises using a contactless reader or a mobile phone device as second device of the second user. The contactless reader is for example connected to or integrated within a PC, laptop, tablet...

The method comprises a step of accessing said information over a near range communication such as NFC, Bluetooth, zigbee, Wi-Fi or any other near range communication technology. It comprises using the first device 1 of the first user for transferring contactlessly said ICE information from the first device 1 of the first user to the second device 2 of the second user.

The method comprises taping the first device 1 of the first user, and fetching said encrypted ICE information with the second device 2 of the second user over the short range communication link. For doing so, when the first user 1 is for example found unconscious after an accident, the second user such as a member of an ambulance or a member of the first rescue team uses the second device 2 such as a contactless reader 2 or a mobile device such as a mobile phone, the contactless reader 2 or the mobile device comprising the private key of the health authority. The second user then taps the first device 1 of the first user. The encrypted ICE information is decrypted with the private key of the health authority.

If the ICE application is installed on the secured element 10 of the first device 1 of the first user, the ICE information related to the first user encrypted with the public key of the health authority is fetched by the contactless reader or the second device 2 of the second user using near range communication such as NFC, Bluetooth, zigbee, Wi-Fi or other near range communication technologies.

Then upon receiving the encrypted data, the second device 2 of the second user applies its private key to the received data and decrypts the first user's sensitive health information, emergency contact. It optionally decrypts health insurance information. The emergency team can then directly contact the patient's next of kin and can be instantly aware of any allergies, blood type of the patient ...

In another embodiment, the ICE information is not encrypted and the health authority can access directly to ICE information. In this case, the first user when registering is aware of which ICE information is readily accessible or not. This is advantageous when the first user visits a foreign country and renders ICE information accessible in case of accident.

In another embodiment, the first user is able to decide which ICE information may be available such as the blood type unsecurely and which ICE information needs a secure transfer such as his/her personal address.

Thanks to this method, it is possible to access easily to sensitive information related to a person. There is no need to enter a PIN code and an authorized person accessing to ICE information is advantageously fast and allows to save person even when unconscious.

## Claims

1. Method for transferring in-case-of-emergency (ICE) information from a first device (1) of a first user to a second device (2) of a second user **characterized in that** it comprises transferring said information with a near range communication.

2. Method for transferring in-case-of-emergency (ICE) information according to claim 1, **characterized in that** it comprises
a. storing said information securely in said first device by the first user, said information being related to said first user and encrypted with the public key of the second user;
b. fetching said encrypted information with the second device over the near range communication.

3. Method according to one of the previous claims, **characterized in that** said information comprises name, medical history, allergies, vaccinations, blood type and/or emergency contact information related to said first user.

4. Method according to one of the previous claim, **characterized in that** the information is signed with the public key of the second user.

5. Method according to one of the previous claims, **characterized in that** the second user is an authorized authority or works for an authorized authority, said authorized authority being a health authority or other government entity needing access to ICE information.

6. Method according to any of the previous claims, **characterized in that** it comprises transferring said information over NFC, Bluetooth, zigbee, or Wi-Fi.

7. Method according to one of the previous claims, **characterized in that** it comprises using a secure element supporting contactless data exchange for storing said ICE information into the first device.

8. Method according to claim 7, **characterized in that** it comprises using an embedded secure element or an NFC-capable UICC, or a MicroSD as secure element.

9. Method according to any of the previous claims, **characterized in that** it comprises using a contactless reader or a mobile phone device as second device.

10. Method according to any of the previous claims, **characterized in that** upon receiving the encrypted information, the second device applies its private key to said information and decrypts said first information.
